(19)

Europäisches
Patentamt
European
Patent Office
Office européen
des brevets

(11) **EP 2 438 849 A1**

(12) **EUROPEAN PATENT APPLICATION**

(43) Date of publication:
**11.04.2012 Bulletin 2012/15**

(51) Int Cl.:
***A61B 5/024*** *(2006.01)*

(21) Application number: **10195042.6**

(22) Date of filing: **15.12.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **06.10.2010 LV 100138**

(71) Applicant: **Latvijas Universitate**
**1586 Riga (LV)**

(72) Inventors:
 • **Erts, Renars**
 **LV-5070 Lielvarde (LV)**

 • **Rubins, Uldis**
 **LV-1063 Riga (LV)**
 • **Upmalis, Vladimirs**
 **LV-5001 Ogre (LV)**
 • **Spigulis, Janis**
 **LV-1050 Riga (LV)**
 • **Svilans, Mikelis**
 **LV-1001 Riga (LV)**

(74) Representative: **Fortuna, Aleksandra**
 **Foral Patent Law Offices**
 **P.O.Box 98**
 **Riga 1050 (LV)**

(54) **Device and method for an optical contactless monitoring of cardiac parameters**

(57)    A contactless photoplethysmographic (PPG) device and method is disclosed for determining heart rate and other parameters simultaneously for several people being in rest and in motion. The proposed device comprises: a radiation-sensitive sensor (20) having the field of view (140) for obtaining electronic images (80) of one or more people (40); the sensor (20) being adapted to monitoring the dynamics of tissue blood volume changes, a distance meter (130) adapted for measuring the distance from the photoplethysmographical device (10) to one or more people (40), a distance meter guidance system (120), adapted for setting and changing the orientation of the meter (130), an output unit (100), adapted for displaying one or more people's (40) heart rate and its derived parameters, a signal processing unit (70) adapted for image (80) processing, analysis and storage, as well as adapted for controlling the system (120).

**Fig. 1**

EP 2 438 849 A1

# EP 2 438 849 A1

**Description**

<u>Technical Field</u>

This invention relates to cardiology and, in particular - to the detection and recording of the human heart rate frequency and other parameters simultaneously to several people being in piece and in motion by using contactless photoplethysmography (PPG) device and method.

<u>Background Art</u>

One of the ways to evaluate a man's heart condition is to measure heart rate frequency, i.e. pulse rate. Heart rate frequency control is essential both in medicine and in preventive health assessment or self-control, and during sports training. Frequent pulse of heart of vascular patients and practically healthy people can be an indicator that shows vegetative nervous system disbalance — sustained increased sympathetic nervous system activity. This disbalance can cause and enhance myocardial ischaemia and stenocardia, reduction of arterial flexibility, the development of atherosclerosis, high blood pressure (hypertension).

It is considered that a normal heart rate frequency of adult person in peace is 60-100 beats per minute. Well-trained athletes at rest can have 40-60 heart beats per minute, but during load it may be even up to 200 beats per minute. Heart rate frequency measurements are also important during sports training, in order to determine load intensity.

In addition to heart rate frequency an important physiological parameter is heart rate variability. Reduced heart rate variability has been observed, for example, to patients with depression, posttraumatic stress syndrome, attention deficit disorder, and practically healthy people during stress and mental load. Pursuant to the heart rate variability it is possible to identify patients at risk after having myocardial infarction and other cardiac conditions (arrhythmia, artery atherosclerosis, heart failure). Quantitative and qualitative parameters of heart rhythm variability (standard deviation, coefficient of variation, etc.) reflect the effect of vegetative nervous system to cardiac sinus node and shows the balance of sympathetic and parasympathetic nervous system and the body's functional reserves for the regulation thereof, and psychoemotional state (E.I. Masterova, V.N. Vasil'ev, T.I. Nevidimova, V.I. Vlasenko. Relation between the psychoemotional state and the heart rate regulation and immune status in human. Ross Fiziol Zh Im I M Sechenova, 85(5):621-7, 1999).

There are several non-invasive heart rate frequency detection methods, such as electrocardiography, ultrasonography, echocardiography, etc. The most widely used method for detection the heart rate frequency is electrocardiography (ECG), which operating principle is based on recording of a heart origin potential difference from the body surface, that reflects the electric potential propagation in the heart. Heart rate is detected by measuring the time between two consecutive ECG R waves and calculating the number of such intervals in one minute (J.R. Hampton. The ECG made easy. International Edition, Edinburgh, Churchill Livingstone, p 5, 1997).

Heart rate variability (periodic fluctuations of cardiac cycle length) is obtained by analyzing the number of successive RR intervals and by getting time between the maximum or minimum values of cardiac cycles. Connection to the human body, as well as quantity of wires connecting the human body with monitoring apparatus makes the use of ECG method in heart rate frequency detection burdensome and limits the mobility of human being observed.

Photoplethysmography (PPG) is an optical method, by which it is possible to non-invasively obtain information on the dynamics of blood volume changes caused by cardiac tissues by using optical radiation. The vascular network is situated under the skin, and because of the heart rate, blood pulsates in blood vessels; according to the periodic changes in the volume, the absorption of the light emitted into tissues changes. By directing the optical radiation to the tissue surface, part of the radiation is reflected, part is dissipated and part is absorbed, therefore from the change in time of the back-scattered optical radiation intensity after the photoelectric conversion an electric PPG signal can be obtained. By using contact probes with specially arranged receivers, it is possible to register these changes in time — the variable (AC) component of the photoplethysmographical signal. The method for measurement of photoplethysmographical signal most often is used in the so-called "therapeutic window" wavelength range from 600 to 1300 nm, where blood and water has the lowest absorption, in order to allow as possible deeper penetration of the optical radiation into tissues and to obtain information from the tissue deeper layers (J. Allen. Photoplethysmography and its application in clinical physiological measurement. Physiological Measurement, Vol. 28, pp. 1-39, 2007). To get the most reflective PPG signal amplitude the red color, or close IR range (650-950 nm) are often chosen as the source of optical radiation, that is an appropriate compromise between the absorption and penetration depth in tissue.

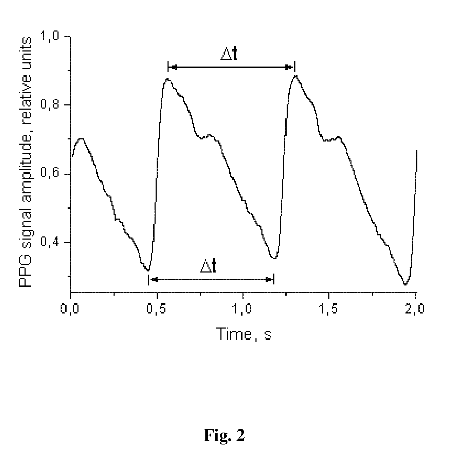

For the heart rate frequency detection the PPG signal could be used by measuring the time ($\Delta t$) between the PPG signal maximum or minimum values of two consecutive pulse periods (Fig. 2) (L. Lindberg, H. Ugnell, P. Oberg. Monitoring of respiratory and heart rates using a fibre-optic sensor. Medical & Biological Engineering & Computing, Vol. 32, pp. 533-537, 1992).

There is known a method and device for the monitoring of physiological parameters (heart rate frequency and

2

variability) by using the contact PPG signal (patent US 7001337 B2), from which the unwanted frequency components are filtered off and further it is processed to obtain the information on the autonomous nervous system, blood pressure changes in human and respiratory rate. This method and the device do not provide PPG signal measurement in a contactless manner and is designed only for one person's measurements. If more people measurements are needed, they are taken in sequence.

There is known a method and device (patent JP 3787336 B2) for the detection of the human heart rate frequency during movement by specially designed signal processing algorithms, using the contact photoplethysmograpical signal. The artifact level detection system is used that monitors what amount of movement at that moment occurs, and then by the wavelet transform detects the pulse rate value. As in the previous method, also here the PPG signal measurement by contactless manner is not provided.

There is known a sensor device for heart rate frequency monitoring by the PPG method (international patent application WO 2009100776 A1), which consists of several parts: a light source, a cell phone, camera operating in video mode, and the processor connected to the camera. The light source may be substituted by natural light. Contacting the device (for instance putting a finger at the camera lens) records the optical radiation that passed through living tissues and performs the image analysis and calculations required. The limitation of this device and method is that the PPG signal is measured only for one person and monitoring of ambient radiation in order to obtain high-quality PPG signal is not provided.

A device for heart rate frequency contactless and contact monitoring is also known (patent application US 2009/0226071 A1), which consists of a cell phone camera, that stores in the device memory both color and monochrome static images, from which the processor selects the red, green and blue (RGB) pixels, which amplitude values are used to obtain the PPG signal, from which the heart rate frequency is being calculated, which can then be further transmitted through a computer network $\sqrt{(R^2 + G^2 + B^2)}$ to any data storing system. Both passed through and back-scattered radiation can be used. To reduce the amount of storable data, a value from each pixel RGB signal values is calculated, that is used for further Fourier mathematical analysis to calculate pulse rate. The device and method does not provide simultaneous contactless PPG signal measurement for several people, nor is able to track the movement of a human. A human must not move during the measurements, since no algorithms are provided for filtering off human movements. The device does not detect whether there is enough light for obtaining high-quality PPG signals.

A study was conducted on differences of contact and contactless PPG signals (K. Humphreys, C. Markham, T.E. Ward. A CMOS camera-based system for clinical photoplethysmographic applications. Proc. SPIE, 5823, pp. 88-95, 2005). An infrared (IR) light emitting diode with a wavelength of 800nm and monochrome CMOS-type video camera with a resolution of $1280 \times 1024$ pixels, connected to a computer via Firewire interface was used in the contactless PPG measurements. Active area of 320x240 pixels and frame frequency of 30 frames/s was used in measurements. Each video frame image was divided into 20x20 pixel areas and the average intensity value for each zone was calculated. PPG signal was measured simultaneously with contactless measurement of PPG signals, using a standard contact pulse oximeter (Nellcor). It was concluded that both contact and contactless PPG waveforms and Fourier spectrums of frequency are very similar. This device does not perform simultaneous PPG signal measurements for several people and does not track the movement of a human. Similarly, only the IR as a light source is used, not the ambient radiation.

A study was conducted on the PPG signal acquisition using natural lighting and a video camera (W. Verkruysse, L.O. Svaasand, J.S. Nelson. Remote plethysmographic imaging using ambient light. Opt. Express, 16(26): 21434—21445, 2008). The video camera was set in manual mode to ensure constant exposure, color and aperture settings. Filming took place for several minutes, with a frame frequency of 15 or 30 frames/s with 320x240 or 640x480 pixel resolution. PPG signal measurement of facial skin with pathological "port-wine stain" syndrome was carried out before and after laser treatment. Performing Fourier analysis for PPG signal, the researchers found that among healthy and pathologic skin a PPG signal phase shift exists. This method does not provide simultaneous PPG signal phase shift measurements of several people and does not track the movement of a human. Therefore a human must not move during the measurements, since no algorithm is provided for measuring PPG signals during movement.

There is known a device for automatic tracking a human via video phone. It consists of two engines, which are able to move the camera up, down, right, left, to follow the human body movement (patent application KR 1020050004572 A) in cases when the software built-in the apparatus detects movements ongoing in the camera's field of view. A mobile phone or Internet connection can be connected to the device that can transmit a report on the movement that occurs in the video phone's field of view. This device does not provide human PPG signal measurements, therefore heart rate information and its derived parameters on human's being under monitoring is not obtained.

There is known a method for human motion tracking, by processing series of successive video images (patent US 5,953,055). Video camera's output is connected to the processing unit which digitizes video images and stores in memory. One or more zones of interest are defined in the video field, where, for example, a man, who fixedly looks at an object of interest, such as cash dispenser, is detected; as well as the number of people within the zone is being determined. This device does not provide for human PPG signal measurement and information on heart rate and its derived parameters are not being obtained.

There is known a device for automatic image focusing for use with digital camera (patent application JP 2010-91709). It consists of an image detection device, a calculation unit and focus drive motor, which controls the device lens system. The detected optical image is converted into digital form; the calculation unit computes image evaluation value. Focus management system, based on the acceleration / deceleration function, conducts the focus drive motor system, until the optimal focus value is reached. This device does not provide human PPG signal measurement and information on heart rate and its derived parameters are not being obtained.

A contactless method and device is known for detection, identifying and analyzing in real time people and other objects, by using video frame detection system in security screening stations, public and other locations to identify the human activities for security and other purposes (patent US 7200266 B2). In case the device detects a new moving or motionless object, it compares the identified objects with the parameters of objects placed in a database, thus providing identification of objects of interest, for example, a man, a bag, a dog, etc. The method also provides tracking of human motions and the identification of motion's type. This method and device does not provide human PPG signal measurements from which the information on the person's heart rate and its derived parameters could be obtained, nor it allows distinguishing or sorting objects of interest by the distance from their location to the device.

The known devices and methods do not provide the opportunity to simultaneous recording in contactless manner both in peace and in motion being humans' heart rate and calculating heart rate parameters (e.g. the current value of heart rate frequency, average heart rate frequency, standard deviation, coefficient of variations, etc.).

Disclosure of Invention

The objective of the invention is to develop a method that provides for simultaneous contactless recording of heart rate and its derived parameters (e.g., average heart rate frequency, standard deviation, coefficient of variations, etc.) of a number of people being both in peace and in motion at exactly defined distance.

There is a method offered by which heart rate frequency and its derived parameters of several people simultaneously could be detected at distance in contactless manner and the results could be shown on a graphical or digital display and stored into data carrier for further processing. Cardiac parameters are being measured only for those people who are at a certain distance. If the calculated heart rate frequency value or its derivative parameters exceeds separately adjustable maximum or minimum value, the system generates an alarm signal. For recording the PPG signal the red (R) value of RGB pixels is preferably used, because red radiation has greater depth of penetration below the skin due to lower subcutaneous blood absorption, so the higher degree of PPG signal amplitude and with a lower noise level is reached than the green or blue radiation gives, from which further heart rate frequency and its derived parameters are being calculated. To create a PPG signal, it is also possible to use certain formulas, for example, formula described in US 2009/0226071 A1. The device for heart rate frequency non-invasive and continuous detection is illustrated in the accompanying drawings.

Brief Description of Drawings

Fig. 1 is a block diagram of the device for heart rate non-invasive contactless detection.
Fig. 2 is an illustration of an example of photoplethysmographic (PPG) signal, consisting of two vascular pulsations, between the maximums or minimums of which time $\Delta t$ can be measured, from which further cardiac parameters can be calculated; the horizontal axis shows the time, the vertical axis shows the photoplethysmographic (PPG) signal amplitude in relative units.
Fig. 3 is a flowchart showing the method for contactless optical cardiac parameters control simultaneously for number of people being in peace and in motion.
Fig. 4 is a flowchart that represents the calculation method of one or more people's movement speed.

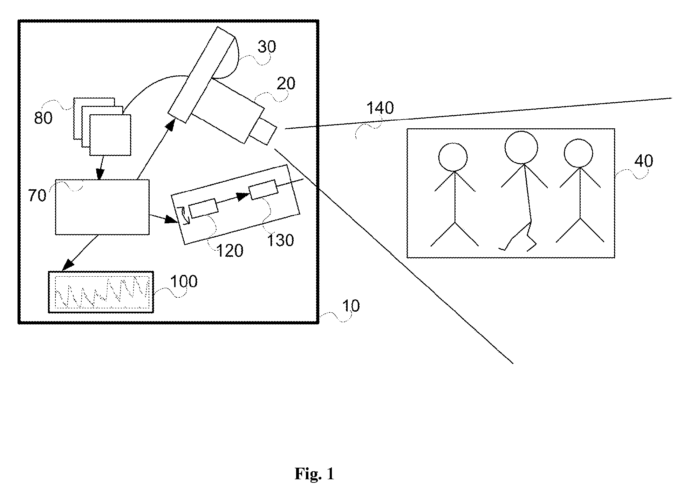

A block diagram of the portable contactless heart rate monitoring device 10 is shown in Fig. 1. The device contains a radiation-sensitive sensor 20, which has a field of view 140, individually switchable on/off radiation source 30, a distance meter 130, the focus of which can be modified by the distance meter's guidance system 120, a signal processing unit 70 which is adapted so that the distance meter's guidance system 120 and a radiation source 30 can be guided through the appropriate connections, as well as the results of signal processing can be displayed at the output unit 100.

In order to make an image record of one or more people 40 being in peace and/or in motion, the radiation-sensitive sensor 20 is equipped with optics which provides the necessary field of view 140.

A number of the successively recorded images 80 can be transmitted from the radiation-sensitive sensor 20 to the signal processing unit 70, which provides their processing, including reading the pixel amplitudes of the radiation-sensitive sensor 20, calculation of combination thereof, detection of the image area in which PPG signals arise, reduction of motion artifact, calculation of heart rate frequency and its derived parameters, signaling of rapid or adverse change of heart rate frequency and/or its derived parameters (e.g., average heart rate frequency, standard deviation, coefficient of variation, etc.).

As the radiation-sensitive sensor 20 a variety of optical receivers may be used, both individual detectors and detector arrays, such as monochrome or color CCD and CMOS cameras which include matrix of pixels. Their sensitivity should be as possible best coordinated with respective surrounding light intensity in the optical spectral area used, for example, the sensitivity of 0.3 Lux at 46 dB signal/noise ratio in the visible (typically 400-760 nm) and in the near infrared (IR) (800 - 1400 nm) field could be reached by the monochrome matrix. Frame frequency of 15 or 30 frames/s and a resolution of 320x240 or 640x480 pixels are widespread. Monochrome sensors' advantage is greater sensitivity, and less processed pixel quantity. Whereas the color sensors (RGB) can get a higher image contrast and thereby improve the signal/noise ratio, but the amount of pixels processed increases thereby imposing additional burdens on the signal processing unit 70.

The spectral area of interest for photoplethysmographical application includes the so-called "therapeutic window" wavelength range 600 - 1300 nm, where subcutaneous blood and fluid has a low absorption, which allows sufficiently deep penetration of optical radiation in the tissues, but at the same time ensures as possible greater PPG signal amplitude. Most advantageous wavelength is in the range of red and short IR (650-950 nm).

The radiation source 30 can be applied in circumstances where the surrounding light intensity is not sufficient to provide the necessary PPG signal quality of the radiation-sensitive sensor 20. It illuminates all the detectable objects in the field of view 140 of the radiation-sensitive sensor 20. For the illumination of objects halogen lamps could be used, visible or infrared light-emitting diodes, or other appropriate devices. The radiation source 30 can operate continuously, but in circumstances where it is needed to reduce the device's power consumption, such as in case of feeding from the battery, radiation can also be pulsating with frequency, which is aligned with the applied frame rate.

Using the higher frame rate, PPG signal integration in time could be used (by shooting, for example, 3 frames and mathematically calculating the average of them), thus improving the signal/noise ratio, at the time it requires more data processing power of the signal processing unit 70.

To ensure distance detection of people being in peace or in motion from the radiation-sensitive sensor 20, the signal processing unit 70 is being connected to the distance measurement guidance system 120, which contains the engine and the distance meter 130, such as laser or ultrasonic range finder. The engine provides direction of the distance meter 130 horizontally (0° - 360°) and/or vertically (0°, 180°).

If the distance between the radiation-sensitive sensor 20 and people 40 that are monitored exceeds a specific, separately adjustable value, the signal processing unit 70 stops monitoring heart rate frequency and derived parameters of the human; if the person returns in the radiation-sensitive sensor's 20 operation area and the signal processing unit 70 identifies the person, his heart rate frequency and its derived parameters' monitoring is being continued. A human's (face and/or height) identification is being performed as follows: the device has a separate operation mode when taking a human's face and/or height images from different angles, and they are stored in the device memory in digital form as mathematical matrix. If the unit has to recognize a human's face or height, the device detects the human outline, converts it into a matrix form and compared with pre-stored template values. The signal processing unit 70 is being connected to the output unit 100 - monitor, digital display, printer, plotter or similar device that provides data imaging.

The calculated heart rate frequency and its derived parameters of each human that is monitored, time of the measurement, date, coordinates of the movement are being stored in separate file, for example, in XML, ASCII or other format. The proposed device may be a standalone unit, but may also consist of several interconnected blocks (20-130).

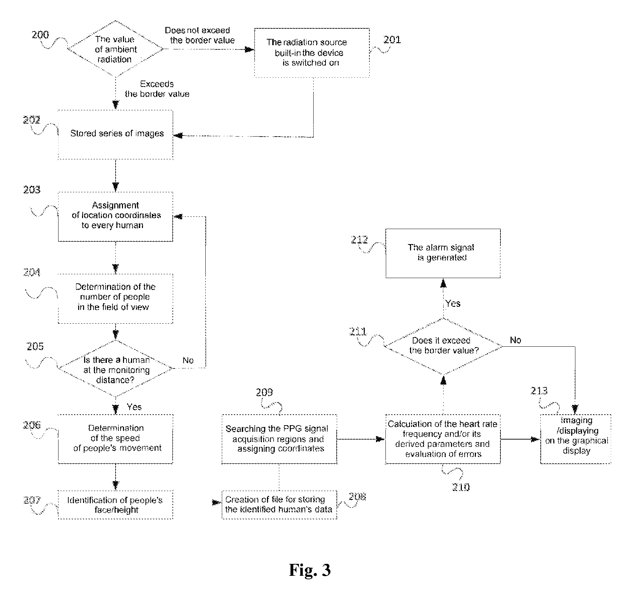

The operation algorithm of the proposed device for general contactless heart rate frequency and its derived value determination is shown in Fig. 3. The ambient radiation value 200 is being measured and, if it exceeds the border value, a number of existing image series 202 are stored, when the ambient radiation is below the border value, the impulsive or continuous radiation source 201 is being switched on. After storage of the initial image series, the location coordinates 203 for every human that is in the device's field of view 140 are being determined, the number of people being in the device's field of view 204 is determined and it is determined whether each identified person that is in the device's field of view is in the distance of monitoring 205 by using remote guidance system 120, if not, then device continues adjusting the coordinates of each person 203 and is searching when human appears at a distance of monitoring 205. If a person is at the monitoring distance 205, where one or more people moving, the determination 206 of the people's movement speed is being carried out. People's face/height identification 207 is being carried out, if a person has not previously been in the "visual field" of the signal processing unit 70 the individual file 208 is being created for that person, if the person has previously been in the field of view of the signal processing unit 70 the data further received are being recorded in this created file. Regions 209 suitable for heartbeat detection are being searched for every human and own coordinates are being adjusted to each such region. From the regions suitable for heartbeat detection heart rate frequency and its derived parameters 210 are being calculated and values exceeding the margin of error are deselected. The calculated parameters are shown in the digital or graphical display 213. If the calculated heart rate frequency value or its derivative parameters exceeds the critical border value 211, the alarm signal 212 is being generated.

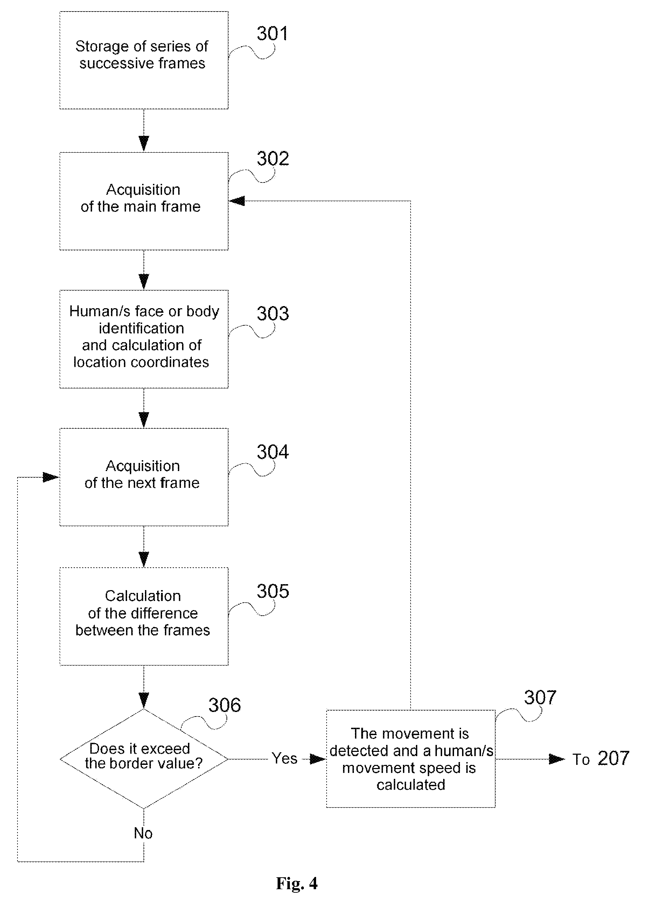

The prior art algorithm for movement detection of one or more humans (Fig. 4) is as follows: successive video images 301 are being stored, the main frame 302 is being taken, from which, it is determined how many people are in the frame using the human height or face identification algorithm, and the location coordinates 303 of each person are being adjusted. Further video frames acquisition 304 continues, location coordinates of every human are being determined, and the difference of location coordinates between the current and previous frames is calculated for every human 305. If the difference exceeds the border value 306, human movement is being detected and speed of human movement is being calculated 307, in turn, if the difference does not exceed the border value, the next frame 302 is obtained, from which further calculations are being made.

Examples of Implementation of the Invention

For contactless measurement of heart rate frequency and its derived parameters the radiation value of all the surrounding is being measured. If it is below the border value, which is required (typically about 500 1x) to obtain high-quality PPG signals, a light source, operating in impulsive or continuous mode is being switched on to ensure high-quality PPG signal acquisition, from which, after Fourier mathematical analysis, it is possible to clearly distinguish heart rate frequency component.

The radiation is being dispersed in the subcutaneous bloodstream and part of it is being absorbed; with the heart rate blood volume changes in a pulsating way and accordingly the back-scattered radiation from the tissues changes. By illuminating living tissues by suitable light source (infrared or visible), the reflected optical signals are being detected by the monochrome or color radiation-sensitive sensor 20.

The processing unit 70 stores successive video images 80, the size of which depends on the number of people, that are in the field of view, and the distance from them to the radiation-sensitive sensor 20, typically 640x480 pixels. Image capture speed must be at least 15 frames per second (the higher it is the greater is the estimated accuracy of heart rate frequency parameters). Video images storage period is at least 15 to 50 seconds, i.e. time that is necessary for optimal detection of heart rate parameters.

The processing unit 70 from the successive video image pixels stores either (i) red, green and blue (RGB) pixel amplitude values in 3 different arrays, or (ii) monochrome radiation-sensitive sensor's 20 pixel amplitude values in one array.

For further processing the radiation sensitive detector's pixel amplitude value or a combination thereof is being selected. The resulting images processing unit 70 processes by using a facial or height identification algorithms.

The processing unit 70 determines how many people are in the device's field of view (the optimal number of people is no more than 5, this is determined by the resolution of the radiation-sensitive sensor 20 and the optics, because in order to obtain high quality PPG signals, a human, who is in the radiation-sensitive sensor's 20 field of view usually has to occupy area of at least $100 \times 100$ pixels so as in further processing high-quality PPG signals would be achieved, where the signal/noise ratio is the highest). For each person in the device's field of view processing unit 70 records the areas in which each person is located by coordinates x, y. By means of distance measurement guidance system 110, processing unit 70 determines what the distance is up to each person. If the distance is greater than separately adjustable value (usually 5 meters), the processing unit 70 removes the human's position coordinates x, y from further processing.

If a human has been found who has not previously been in the device's field of view, the processing unit 70 creates a profile (e.g., file) for the human, where the human's image and further human's cardiac parameters are being stored. If a human has been found who has previously been in the device's field of view, the device receives information about the identified human from the memory and further calculated heart rate parameters are being recorded in the present human's profile.

From areas of identified people the processing unit 70 determines heart rate frequency detectable regions in the following way: from the pixel arrays values of the stored video images or combinations thereof typical frequencies of 0.66 to 3 Hz are being searched in the spectral area of the frequency by means of Fourier mathematical analysis, because the heart rate frequency in the Fourier frequency distribution is clearly marked and with at least twice greater amplitude than the other frequency components.

Each region suitable for heart rate frequency detection (usually at least 10x10 pixels) which size depends on possibility to ensure the sufficiently high signal / noise ratio, is being assigned by own coordinate x, y. From the video signal images' regions suitable for heart rate frequency detection, the processing unit 70 calculates heart rate frequency for each human recognized, using the following methods:

1) using Fourier mathematical analysis, searching in the distribution range for the frequency of 0.66 to 3 Hz, wich has the maximum amplitude, and using the formula

$$heart\ rate\ frequency = 60 * frequency\ of\ maximum\ amplitude,$$

2) from the video images pixels, for example, the pixel value combinations are being selected and the maximum or minimum values of PPG signal are being searched, by measuring the time ($\Delta$t) between two consecutive pulse period maximum or minimum values in the photoplethysmographical signal (Fig. 2) and using the formula

$$f = \frac{60}{\Delta t} \qquad (2).$$

In order to improve the accuracy of heart rate frequency calculation, the processing unit 70 compares the difference of values of both heart rate frequency detection methods: if they differ for more than 10%, they are excluded from further processing. Similarly, if one human's heart rate frequency values from different regions suitable for heart rate frequency determination differ by 10%, they are excluded from further processing.

The average heart rate frequency for each human being in the device's field of view is determined by using the formula:

$$\bar{f} = \frac{1}{n}\sum f_i \qquad (3),$$

where $f_i$ - heart rate frequency current value, $\bar{f}$ - the average heart rate frequency value, n - the calculated heart rate frequency range.

Heart rate frequency standard deviation $\sigma$ for each human being in the device's field of view is being determined using the formula:

$$\sigma = \sqrt{\frac{1}{n}\sum(f_i - \bar{f})} \qquad (4).$$

Heart rate frequency variation coefficient for each human being in the device's field of view is being determined using the formula:

$$c_v = \frac{\sigma}{\bar{f}} \qquad (5).$$

If any human's heart rate frequency or its derived parameter exceeds a separately adjustable critical minimum or maximum value (for example, 180 beats per minute or 40 beats per minute), the processing unit 70 generates a warning or alarm signal.

From video signal images the processing unit 70 calculates, whether the identified people move and with what speed (Fig. 4), the optimal speed <6 km / hour, that is determined by the speed of the radiation-sensitive sensor's 20 image acquisition and processing of the processing unit 70.

The processing unit 70 reproduces the calculated heart rate parameters in a graphical or digital display 10 in the real time mode.

The second contactless heart rate frequency and its derived values determining method is similar to the previously described, with the difference that the processing unit 70 on the graphic display in real time mode reproduces an image obtained by the radiation-sensitive sensor 20, and next to each identified human at the distance of some graphic screen pixels also reproduces heart rate frequency or its derived values.

The described invention was used for monitoring heart rate frequency for people who exercised in the gym. The gym's lighting is 600 lux, which is sufficient to get the PPG signals, which signal/noise ratio is the highest. When the first person enters the gym (dimensions 7x10 m) and begins to take exercises at the distance of 5 meters from the contactless recording apparatus with the built-in monochrome radiation-sensitive sensor (having sensitivity of 0.3 Lux, signal/noise ratio 46 dB), the processing unit records the movement and establishes, that there is a human in the radiation-sensitive sensor's field of view, who has not previously been in the field of view. A new data profile is being created for the human who is identified for the first time. It is detected, that the human naked areas - forehead, palms of both hands and one shin is suitable for registration of the heart rate frequency and its derived parameters, from these regions cardiac parameters are started to be registered and they are being recorded in the created human's data profile. It is detected, that the regions suitable for registration of the heart rate frequency and its derived parameters are the human's both thighs, from these regions cardiac parameters are started to be registered and they are being recorded in the identified human's profile. Continuing the exercises, the human's heart rate frequency rapidly changes from 115 to 40 beats/min, the apparatus generates an alarm signal.

The proposed device compared to other devices is easier in use — it is non-invasive and its location does not restrict the movement, it automatically determines whether the ambient radiation is sufficient to obtain qualitative and treatable PPG signals. It is possible to get heart rate frequency and its derived values simultaneously for several people being in peace and in motion.

The height or face recognition system is being built into the device to identify human and get the history of changes of his heart rate frequency and its derivative parameters. The device can be used in conjunction with any other body's physiological state investigation techniques.

The device is lightweight, so it can be integrated into other devices, as well as the supply voltage can be obtained from rechargeable batteries or solar cells.

The proposed device has a wide range of applications such as for diagnostics in medical institutions, for monitoring physiological parameters of infants in incubators and sleeping people; in patients with skin burns, in vehicles, outdoors and indoors in fitness halls, during sports competitions and mass events, for safety control systems at airports (for example, for contactless monitoring of rapid heart rate frequency change in value of the investigated suspicious person approaching the security control), etc.

**Claims**

1. A photoplethysmographical device (10), comprising:

   - a radiation-sensitive sensor (20) having a field of view (140) for obtaining an electronic image (80) of one or more people (40), where the sensor (20) is adapted for monitoring of the dynamics of the tissue blood volume changes caused by function of the heart,
   - a distance meter (130), adapted for measuring the distance from the photoplethysmographical device (10) to one or more people (40),
   - a distance meter guidance system (120), which is adapted for setting and changing the orientation of the meter (130),
   - an output unit (100), which is adapted for imaging or displaying of electronic information and/or signaling on one or more people (40) heart rate frequency and its derived parameters;
   - a signal processing unit (70) being connected with the meter (130), the system (120), the sensor (20) and the output unit (100) with ability to transmit and/or exchange the signals and is adapted for image (80) processing, analysis and storage, as well as adapted for controlling the system (120) and imaging or displaying the signal processing results on the output unit (100).

2. The device according to claim 1, **characterized in that** the radiation-sensitive sensor's (20) sensitive spectral area wavelength is between 600 nm and 1300 nm, preferably between 650 nm and 950 nm.

3. The device according to claim 1 or 2, **characterized in that** the radiation-sensitive sensor (20) is being selected from the group consisting of a CMOS color camera, color CCD camera and monochrome picture camera.

4. The device according to any of the previous claims, **characterized in that** it further comprises a source of radiation (30) being controllable via the signal processing unit (70); the source of radiation (30) being adapted for illumination of one or more people (40) that are in the sensor's (20) field of view (140) with a wavelength being in the sensor's (20) sensitive spectral area.

5. The device according to any of the previous claims, **characterized in that** the distance meter (120), the sensor (20) and the source of radiation (30) are being arranged with ability to be directed horizontally 0°-360° and/or vertically 0°-180°.

6. A method for monitoring the heart rate frequency and its derivative parameters, comprising the following steps:

   i) installation of a radiation-sensitive sensor (20) having a field of view (140) and orientation of the field of view (140) to potential location of people (40);
   ii) electronic acquisition of images (80) by the radiation-sensitive sensor (20) from the field of view (140) and storage thereof in a signal-processing unit (70);
   iii) recording of each human's location area on the electronically acquired images (80), assigning them coordinates x, y, using the processing unit (70);
   iv) determination of the opened body areas in the location areas of each human, being in the field of view (140);
   v) collection of a number of consistent photoplethysmographic signal data reflected from the opened human body's areas by the signal processing unit (70);
   vi) registration of the collected data of photoplethysmographic signals;
   vii) calculation of the heart rate frequency and/or its derived parameter, error evaluation and deselection of values, which exceed the margin of error.

7. The method according to claim 6, **characterized in that** the electronically obtained images (80) are being processed by the processing unit (70) using face and/or height identification algorithms.

8. The method according to claim 6 or 7, **characterized in that** after identification of human's face and/or height, the processing unit (70) stores data of each identified human by in the respective file.

9. The method according to any claim from 6 to 8, **characterized in that** the processing unit (70) calculates every identified human's heart rate frequency and/or its derivative parameters by measuring the distance between two photoplethysmographical maximums or minimums from the opened body areas in the electronically obtained images (80).

10. The method according to any claim from 6 to 9, **characterized in that** from the opened body areas in the electronically obtained images (80), the processing unit (70) calculates every identified human's heart rate frequency and/or its derivative parameters using the Fourier frequency analysis.

11. The method according to claim 9 and 10, **characterized in that** if the heart rate frequency calculated by the processing unit (70) is different from the heart rate frequency being obtained by the Fourier frequency analysis by more than 10%, they are excluded from further processing.

12. The method according to any claim from 6 to 11, **characterized in that** after the registration of each human's location area being in the field of view (140), assigning them by coordinates, the processing unit (70) determines the speed of one or more people's movement, controlling the radiation-sensitive sensor's (20) field of view (140), in order to follow one or more people in motion.

13. The method according to any claim from 6 to 12, **characterized in that** after detecting people in the detection distance, the distance to each human being in the field of view (140) is being determined.

14. The method according to claim 13, **characterized in that** the distance to each human being in the field of view (140) is being calculated by the processing unit (70) by measuring the distance from the distance meter (130) to each human being in the field of view (140).

15. The method according to any claim from 6 to 14, **characterized in that** if any human's heart rate frequency or its derived parameter exceeds a pre-set critical minimum or maximum value, the processing unit (70) generates a warning or alarm signal.

**Fig. 1**

**Fig. 2**

200 — The value of ambient radiation

Does not exceed the border value →

201 — The radiation source built-in the device is switched on

Exceeds the border value

202 — Stored series of images

203 — Assignment of location coordinates to every human

204 — Determination of the number of people in the field of view

205 — Is there a human at the monitoring distance? — No

206 — Yes — Determination of the speed of people's movement

207 — Identification of people's face/height

209 — Searching the PPG signal acquisition regions and assigning coordinates

208 — Creation of file for storing the identified human's data

210 — Calculation of the heart rate frequency and/or its derived parameters and evaluation of errors

211 — Does it exceed the border value? — No

212 — The alarm signal is generated

Yes

213 — Imaging /displaying on the graphical display

**Fig. 3**

12

```
┌─────────────────────┐
│  Storage of series of │  ⟋301
│  successive frames    │
└─────────────────────┘
           │
           ▼
┌─────────────────────┐
│    Acquisition        │  ⟋302
│  of the main frame    │◄──────────────┐
└─────────────────────┘                │
           │                           │
           ▼                           │
┌─────────────────────┐                │
│  Human/s face or body │  ⟋303         │
│    identification     │               │
│   and calculation of  │               │
│  location coordinates │               │
└─────────────────────┘                │
           │                           │
           ▼                           │
┌─────────────────────┐                │
│    Acquisition        │  ⟋304         │
│  of the next frame    │◄──┐           │
└─────────────────────┘   │           │
           │               │           │
           ▼               │           │
┌─────────────────────┐    │           │
│    Calculation        │  ⟋305        │
│  of the difference    │    │          │
│  between the frames   │    │          │
└─────────────────────┘    │          │
           │                │          │
           ▼                │          │
         ╱306              │          │
     ╱Does it exceed╲──Yes──►┌──────────────────┐
     ╲the border value?╱     │ The movement is  │ ⟋307
         ╲      ╱            │detected and a human/s│──► To 207
           │                 │ movement speed is    │
          No                 │   calculated         │
           └─────────────────┴──────────────────┘
```

**Fig. 4**

13

Europäisches
Patentamt

European
Patent Office

Office européen
des brevets

# EUROPEAN SEARCH REPORT

Application Number

EP 10 19 5042

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MING-ZHER POH ET AL: "Non-contact, automated cardiac pulse measurements using video imaging and blind source separation", OPTICS EXPRESS, vol. 18, no. 10, 7 May 2010 (2010-05-07), page 10762, XP55016649, ISSN: 1094-4087, DOI: 10.1364/OE.18.010762 | 6-8,10, 12 | INV. A61B5/024 |
| Y | * page 10764, paragraph 4 - page 10765, paragraph 1 * <br> * page 10766, paragraph 2 - page 10767, paragraph 3 * <br> * page 10768, last paragraph - page 10774, paragraph 1 * <br> * figures 1-7 * <br> ----- | 1-5,9, 11,13-15 | |
| Y | US 2004/232317 A1 (URA HARUO [JP] ET AL) 25 November 2004 (2004-11-25) <br> * paragraph [0003] - paragraph [0012] * <br> * paragraph [0028] - paragraph [0037] * <br> * paragraph [0053] - paragraph [0055] * <br> * paragraph [0115] * <br> * figures 1,6 * <br> ----- | 1-5,13, 14 | |
| Y | JIA ZHENG ET AL: "A remote approach to measure blood perfusion from the human face", PROCEEDINGS OF SPIE, vol. 7169, 1 January 2009 (2009-01-01), pages 716917-1-716917-7, XP55016740, ISSN: 0277-786X, DOI: 10.1117/12.807354 <br> * pages 716917-1, paragraph 2 - pages 716917-5, paragraph 2 * <br> * figures 1-4 * <br> ----- <br> -/-- | 9,11 | TECHNICAL FIELDS SEARCHED (IPC) <br><br> A61B <br> G08B |

The present search report has been drawn up for all claims

| Place of search | Date of completion of the search | Examiner |
|---|---|---|
| Berlin | 18 January 2012 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................................

& : member of the same patent family, corresponding document

EPO FORM 1503 03.82 (P04C01)

Europäisches Patentamt
European Patent Office
Office européen des brevets

# EUROPEAN SEARCH REPORT

Application Number

EP 10 19 5042

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DE 10 2004 030216 A1 (DIAPLAN STAHL & HOLZ INNENAUSB [DE]) 5 January 2006 (2006-01-05) * paragraph [0019] * | 15 | |
| E | WO 2011/021128 A2 (KONINKL PHILIPS ELECTRONICS NV [NL]; JEANNE VINCENT [NL]; KIRENKO IHOR) 24 February 2011 (2011-02-24) * page 3, line 22 - page 4, line 26 * * page 6, line 33 - page 7, line 15 * * page 9, line 32 - page 12, line 4 * * page 15, line 10 - page 16, line 30 * * figure 5 * | 6 | |
| A | JP 61 221731 A (MITSUBISHI ELECTRIC CORP) 2 October 1986 (1986-10-02) * abstract * | 1 | |
| A | EP 0 445 334 A1 (SIEMENS AG [DE]) 11 September 1991 (1991-09-11) * column 2, line 29 - column 3, line 4 * * column 4, line 9 - column 6, line 19 * * figure 2 * | 1 | TECHNICAL FIELDS SEARCHED (IPC) |
| A | US 5 473 368 A (HART FRANK J [US]) 5 December 1995 (1995-12-05) * column 6, line 15 - line 41 * * column 7, line 67 - column 9, line 4 * * figures 1,2 * | 1 | |

The present search report has been drawn up for all claims

| Place of search | Date of completion of the search | Examiner |
|---|---|---|
| Berlin | 18 January 2012 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 10 19 5042

This annex lists the patent family members relating to the patent documents cited in the above-mentioned European search report.
The members are as contained in the European Patent Office EDP file on
The European Patent Office is in no way liable for these particulars which are merely given for the purpose of information.

18-01-2012

| Patent document cited in search report | Publication date | Patent family member(s) | | Publication date |
|---|---|---|---|---|
| US 2004232317 A1 | 25-11-2004 | EP 1480006 A2<br>JP 2004340880 A<br>US 2004232317 A1 | | 24-11-2004<br>02-12-2004<br>25-11-2004 |
| DE 102004030216 A1 | 05-01-2006 | NONE | | |
| WO 2011021128 A2 | 24-02-2011 | NONE | | |
| JP 61221731 A | 02-10-1986 | NONE | | |
| EP 0445334 A1 | 11-09-1991 | NONE | | |
| US 5473368 A | 05-12-1995 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7001337 B2
- JP 3787336 B
- WO 2009100776 A1
- US 20090226071 A1
- KR 1020050004572 A
- US 5953055 A
- JP 2010091709 A
- US 7200266 B2

**Non-patent literature cited in the description**

- **ROSS FIZIOL.** *Zh Im I M Sechenova,* 1999, vol. 85 (5), 621-7
- **J.R. HAMPTON.** The ECG made easy. Churchill Livingstone, 1997, 5
- **J. ALLEN.** Photoplethysmography and its application in clinical physiological measurement. *Physiological Measurement,* 2007, vol. 28, 1-39
- **L. LINDBERG ; H. UGNELL ; P. OBERG.** Monitoring of respiratory and heart rates using a fibre-optic sensor. *Medical & Biological Engineering & Computing,* 1992, vol. 32, 533-537
- **K. HUMPHREYS ; C. MARKHAM ; T.E. WARD.** A CMOS camera-based system for clinical photoplethysmographic applications. *Proc. SPIE,* 2005, vol. 5823, 88-95
- **W. VERKRUYSSE ; L.O. SVAASAND ; J.S. NELSON.** Remote plethysmographic imaging using ambient light. *Opt. Express,* 2008, vol. 16 (26), 21434-21445